Europäisches Patentamt

European Patent Office

Office européen des brevets

(19)

(11) Veröffentlichungsnummer: **0 005 737**
**B1**

(12) # EUROPÄISCHE PATENTSCHRIFT

(21) Anmeldenummer: **79101322.0**

(22) Anmeldetag: **02.05.79**

(51) Int. Cl.³: **C 07 C 69/74,**
**C 07 C 67/303,**
**B 01 J 21/04**

(54) **Verfahren zur Herstellung von cycloaliphatischen Carbonsäureestern**

(30) Priorität: **26.05.78 DE 2823165**

(43) Veröffentlichungstag der Anmeldung:
**12.12.79 Patentblatt 79/25**

(45) Bekanntmachung des Hinweises auf die Patenterteilung:
**07.01.81 Patentblatt 81/01**

(84) Benannte Vertragsstaaten:
**BE CH DE FR GB IT NL**

(56) Entgegenhaltungen:
**DE - A - 1 226 567**
**GB - A - 1 013 018**
**US - A - 3 027 398**

(73) Patentinhaber: **BAYER AG**
**Zentralbereich Patente, Marken und Lizenzen**
**D - 5090 Leverkusen 1, Bayerwerk (DE)**

(72) Erfinder: **Halcour, Kurt, Dr.**
**Gellertstrasse 14**
**D - 5090 Leverkusen (DE)**
**Losacker, Paul, Dr.**
**Johannisberg 11**
**D - 5653 Leichlingen 1 (DE)**
**Waldmann, Helmut, Dr.**
**Carl-Rumpff-Strasse 59**
**D - 5090 Leverkusen (DE)**
**Schwerdtel, Wulf, Dr.**
**Hegelstrasse 9**
**D - 5090 Leverkusen (DE)**

Courier Press, Leamington Spa, England.

# 0 005 737

Verfahren zur Herstellung von cycloaliphatischen Carbonsäureestern

Die vorliegende Erfindung betrifft eine kontinuierliches Verfahren zur Hydrierung von aromatischen Carbonsäureestern zu den entsprechenden cycloaliphatischen Carbonsäureestern mit Hilfe eines in einem Festbett angeordneten Nickel- und/oder Ruthenium- und/oder Rhodium- und/oder Palladiumkatalysators, der auf einen Träger aufgebracht ist, welcher zumindest teilweise aus Lithium-Aluminium-Spinell besteht.

Es ist bereits bekannt, Ester der verschiedenen isomeren Phthalsäuren zu den entsprechenden isomeren cycloaliphatischen Hexahydrophthalsäureestern zu hydrieren. Dabei verwendet man ausschließlich die Suspensionskatalyse, d.h., der Katalysator wird in suspendierter Form mit dem zu hydrierenden aromatischen Ester gemischt, teilweise unter Zusatz eines Verdünnungsmittels.

In den meisten Fällen wird Nickel, aufgebracht auf einen Träger, als Katalysator in Suspension verwendet, so z.B. in der US-PS 2.070.770 und der DE-AS 11 54 096. In anderen Fällen benutzt man bei der Suspensionskatalyse Ruthenium als Katalysator, aufgebracht auf Aluminiumoxid oder Aktivkohle (vergl. beispielsweise DE-OS 21 32 547 und US-PS 3.027.398). Gemäß der vorgenannten US-PS 3.027.398 erfolgt die Hydrierung diskontinuierlich innerhalb der gleichen Temperatur- und Druckbereiche wei beim Verfahren der vorliegenden Anmeldung. Nachteilig bei diesem Verfahren ist das erforderliche Abtrennen des Katalysators, der dabei leicht auftretende Katalysatorverlust, die zeitlich relativ begrenzte Aktivität des Katalysators und noch nicht optimale Ausbeuten an reinem Endprodukt.

Prinzipiell ist es auch bekannt, außer Nickel und Ruthenium auch Platin, Palladium und Rhodium als Katalysatoren bei der Kernhydrierung von aromatischen Verbindungen einzusetzen (vergl. "Survey of Organic Syntheses" von G.A. Buehler und O.E. Pearson, Wiley-Interscience 1970, S. 11).

Schließlich ist der GB-PS 1.013.018 zu entnehmen, daß für die kontinuierlich Kernhydrierung von Benzoesäure ein nach speziellem Verfahren hergestellter Nickelkatalysator auf Silica Gel, angeordnet im Festbett, gut geeignet ist. Dagegen würde Nickel auf $\gamma$-Aluminiumoxid zwar einen aktiven Katalysator ergeben, der jedoch nicht genügend mechanisch stabil ist und zu Verstopfungen des Reaktors führt. Seine Lebensdauer ist gering, ebenso wie seine Selektivität (vergl. GB-PS 1.013.018, Seite 4, Zeile 24).

Der Nachteil der bisher beschriebenen Verfahren zur Hydrierung von aromatischen Carbonsäureestern zu den entsprechenden cycloaliphatischen Carbonsäureestern besteht vor allem in der diskontinuierlichen Suspensionskatalyse. Das erfordert ein Eintragen des Katalysators in die Einsatzmischung und ein Abtrennen des Katalysators von der Reaktionsmischung. Diese Abtrennung des Katalysators ist bei den meist pyrophoren Raney-Nickel-Katalysatoren nur unter Beachtung von Vorsichtsmaßnahmen möglich.

Bei der Abtrennung der preislich sehr teuren, suspendierten Edelmetallkatalysatoren (Ru oder Rh) kommt der wirtschaftliche Verlust durch die nicht quantitative Rückgewinnung hinzu. Außerdem lassen sich erfahrungsgemäß einmal benutzte, suspendierte Edelmetallkatalysatoren nur in begrenztem Maße in den Hydrierprozeß zurückführen. Die beschriebenen Nachtiele des notwendigen Katalysatoren ein- und -austrags sind nicht nur auf die diskontinuierliche Sumpfphasehydrierung beschränkt, sondern treten auch bei kontinuierlicher Handhabung des Verfahrens auf.

Ein weiterer Nachteil ist der oft beachtliche Zeitaufwand bei der Sumpfphasehydrierung von aromatischen Carbonsäureestern. So werden z.B. in J. Amer. chem. Soc. 82 (1960), 2547—53; 3—4 h und gemäß J. prakt. Chemie 29 (1965), 235—36 sogar 16 h für die Hydrierung benötigt. In den meisten der beschriebenen Hydrierverfahren wird der zu hydrierende aromatische Ester in Verdünnung mit einem Fremdlösungsmittel (Methanol, Ethanol, Dioxan) hydriert. Von Nachteil dabei ist die erforderliche anschließende destillative Abtrennung des Lösungsmittels.

Aufgabe der vorliegenden Erfindung war es, ein kontinuierliches Verfahren zur Hydrierung von aromatischen Carbonsäureestern zu den entsprechenden cycloaliphatischen Carbonsäureestern aufzufinden, das erlaubt, hohe Raum-Zeit-Ausbeuten zu erzielen, und das auf die Abtrennung suspendierten Katalysators verzichtet. Das Verfahren sollte einen hohen Grad an Wirtschaftlichkeit erbringen, sowie einfach und problemlos durchführbar sein. So sollte das Arbeiten mit pyrophoren Katalysatoren und Katalysatorverluste vermieden werden, und die Menge an Edelmetallkatalysator niedrig gehalten werden können, sowie der Katalysator seine Wirksamkeit ohne besondere Aktivierung über längere Zeiträume behalten. Natürlich sollte das Hydrierprodukt auch eine möglichst hohe Reinheit aufweisen.

Die Aufgabe wurde dadurch gelöst daß ausgewählte Katalysatoren eingesetzt wurden, und die Hydrierung in der Riesel-, Flut- oder Gasphase über in einem Festbett angeordneten Ni- und/oder Ru- und/oder Rh- und/oder Pd-Katalysator, der auf einem Träger aus Aluminiumoxid aufgebracht ist, das zu mindestens 20% in Lithium-Aluminium-Spinell (Li $Al_5O_8$) umgewandelt worden is, durchgeführt wird. Optimale Resultate werden bei der kontinuierlichen Hydrierung erreicht, wenn Ru-, Rh- oder Pd-, insbesondere aber Pd-Katalysatoren eingesetzt werden. Der zu hydrierende Carbonsäureester kann mit dem hydrierten Endprodukt verdünnt werden.

Gegenstand der Erfindung ist somit ein Verfahren zur kontinuierlichen katalytischen Hydrierung von aromatischen Carbonsäureestern zu den entsprechenden cycloaliphatischen Carbonsäureestern,

# 0 005 737

gegebenenfalls in Gegenwart des Hydrierprodukts als Verdünnungsmittel, in der Riesel- oder Flutphase bei Temperaturen von 70°C bis 250°C und Drucken von 30 bar bis 200 bar bzw. in der Gasphase bei 70°C bis 250°C und 1 bar bis 10 bar in Gegenwert von mindestens einem, auf Trägern aufgebrachten Katalysator aus der Reihe Nickel, Ruthenium, Rhodium oder Palladium im Festbett, dadurch gekennzeichnet, daß man als Träger für die Katalysatoren ein Aluminiumoxid verwendet, das zu mindestens 20% in Lithium-Aluminium-Spinell umgewandelt worden ist.

Als aromatische Carbonsäureester können die Ester von aromatischen Mono-, Di-, Tri- und Tetracarbonsäuren eingesetzt werden. Der Aromatenteil kann ein- und mehrcyclisch sein, wobei der aromatische Ring auch Alkylreste mit 1—4 C-Atomen enthalten kann.

Bevorzugt wird das erfindungsgemäße Verfahren angewandt für die Kernhydrierung von Estern der Benzoesäure, O-, m-, p-Toluylsäure, o-Phthalsäure, Isophthalsäure, Terephthalsäure und Homophthalsäure. Als Alkoholkomponente der Ester der aromatischen Carbonsäuren eignen sich Alkohole von $C_1$ bis $C_8$, bevorzugt Methanol und Ethanol.

Die Ester der aromatischen Carbonsäuren können in unverdünnter Form oder unter Zusatz des Hydrierungsproduktes als Verdünnungsmittel kernhydriert werden.

Bei Verwendung eines Verdünnungsmittels kann das Gewichtsverhältnis von zu hydrierender Komponente zum Verdünnungsmittel 1:1 bis 1:100, bevorzugt 1:5 bis 1:30 betragen.

Als Hydriergase können Wasserstoff oder Wasserstoff enthaltende Gase verwendet werden, wobei enthaltene Katalysatorgifte vorher zu entfernen sind. Nach der Reaktion kann ein kleiner Anteil des Restgases entspannt werden, um die Anreicherung von Inertgasen zu vermeiden.

Die Hydrierung in der flüssigen Phase wird bei Drucken oberhalb von 30 bar durchgeführt; im allgemeinen werden Drucke von 50 bis 200 bar, vorzugsweise von 80 bis 150 bar angewendet. Bei der Gasphasenhydrierung werden Drucke von 1 bis 10 bar angewendet, vorzugsweise wird jedoch in der Nähe des Atmosphärendruckes gearbeitet.

Die Temperaturen für die Hydrierung der aromatischen Carbonsäureester sollten 70 bis 250°C betragen, bevorzugt wird ein Temperaturbereich von 120 bis 180°C.

Als Katalysatoren sind die Metalle aus der Reihe Nickel, Ruthenium, Rhodium, Palladium geeignet. Bevorzugt werden Ruthenium, Rhodium oder Palladium, insbesondere Palladium. Es können auch Kombinationen der vorgenannten Metalle eingesetzt werden.

Die Katalysatoren sind mit einem Gehalt von 0,1 bis 5 Gew.-% auf den Katalysatorträger aufgebracht. Als Katalysatorträger eignet sich Aluminiumoxid, das zu mindestens 20% in Lithium-Aluminium-Spinell umgewandelt worden ist. Ein Träger mit einem Gehalt von 70—100% Lithium-Aluminium-Spinell liefert die besten Ergebnisse. Der Katalysatorträger kann als Zylinder, Extrudat, Pellet oder stückig eingesetzt werden, bevorzugt ist jedoch die Kugelform (Durchmesser ca. 1 bis 8 mm).

Der im Festbett angeordnete Katalysator kann in einen Schachtofen eingefüllt sein, wegen der besseren Wärmeabfuhr ist es jedoch vorteilhaft, den Katalysator in einen Rohrbündelreaktor einzufüllen, wobei um die Rohre ein Wärmeabfuhrmedium geleitet wird.

Die Durchführung der Hydrierung kann in der Gas-, Rieseloder Flutphase erfolgen, bevorzugt wird in der Rieseloder Flutphase, insbesondere in der Rieselphase hydriert. Bei der Gasphasehydrierung wird die zu hydrierende Verbindung durch das Hydriergas verdampft, das Hydrierprodukt aus dem Reaktionsgas abgeschieden; ein Teil des Abgases kann in den Hydrierprozeß als Kreisgas zurückgeführt werden. Erfolgt die Hydrierung in der Rieselphase, so rieselt das zu hydrierende Produkt im Reaktor von oben nach unten über den Katalysator, der sich in einer Wasserstoffatmosphäre befindet; der Wasserstoff kann von oben oder von unten in den Reaktor eingeführt werden. Einen Teil des Abgases entspannt man nach Abtrennung des flüssigen Produktes ins Freie. Führt man die Hydrierung in der Flutphase durch, so durchströmen Einsatzprodukt und Wasserstoff, meist in gelöster Form, das Katalysatorbett von unten nach oben.

Die Raum-Zeit-Ausbeute kann 0,1 bis 2 kg an cycloaliphatischem Ester pro Liter Katalysator und Stunde betragen, vorzugsweise unter 1 kg/1 × h.

Das erfindungsgemäße Verfahren wird kontinuierlich durchgeführt.

Die erfindungsgemäß erhaltenen Produkte können als Weichmacher für Kunststoffe sowie als Zwischenprodukte für die Herstellung von Ausgangsstoffen für Epoxidverbindungen eingesetzt werden.

Das erfindungsgemäß Verfahren wird durch die nachfolgenden-Beispiele näher erläutert. Die angegebenen Prozentgehalte beziehen sich auf das Gewicht, sofern nicht anders vermerkt.

*Beschreibung der Hydrierapparatur*

Als Reaktor diente ein 4 bzw. 6 m langes Rohr mit einer lichten Weite von 24 mm. Das Reaktionsrohr (Reaktor) war mit einem Doppelmantel versehen, welcher mit Dampf geheizt und mit Kondensat gekühlt werden konnte. Der Katalysator war in dem Reaktionsrohr fest angeordnet. Über den Katalysator wurde *im Falle der Rieselphasehydrierung* das zu hydrierende Einsatzprodukt, in den meisten Fällen verdünnt mit rückgeführtem Hydrierprodukt, von oben aufgegeben. Am unteren Ende des Reaktors befand sich ein Abscheider, in dem das flüssige Reaktionsprodukt vom Restgas abgeschieden wurde. Meist wurde ein Teil des Reaktionsproduktes zum Einsatz zurückgepumpt, das restliche Reationsprodukt wurde ins Freie entspannt. Der Wasserstoff wurde gemeinsam mit dem flüssigen Einsatzprodukt am oberen Ende des Reaktors unter Druck aufgegeben, währen aus dem unter dem

3

Reaktor befindlichen Abscheider eine kleine Menge von durchweg 10% des theoret, benötigten Wasserstoffgases als Abgas entspannt wurde.

Bei der Durchführung der *Hydrierung in der Flutphase* wurde der gleiche Reaktor benutzt wie bei der Rieselphasehydrierung. Wasserstoff und zu hydrierende Komponente (meist verdünnt mit dem Hydrierprodukt als Verdünnungsmittel) wurden am unteren Ende des Reaktors unter Druck eingeführt. Am oberen Ende Des Reaktors befand sich der Abscheider zur Trennung von Gas- und Flüssigphase, aus dem wie bei dem Abscheider der Rieselphase die Produkte entspannt wurden.

Wurde die Hydrierung in der Gasphase durchgeführt, so geschah dies im gleichen Reaktor, wie er bei der Reiseloder Flutphasehydrierung benutzt wurde. Die zu hydrierende Komponente wurde — verdünnt oder meist unverdünnt in einem Verdampfer (Blasensäule oder Fall- filmverdampfer) mit einem Übershcuß an Wasserstoff verdampft. Das Gasgemisch durchströmte den Reaktor von unten nach oben. Nach Verlassen des Reaktors wurde das Reaktionsprodukt aus dem Abgasstrom durch Quenchung mit schon kondensiertem Hydrierprodukt und/oder Tiefkühlung in die flüssige Form übergeführt. Das Abgas wurde entspannt, es kann aber auch als Kreisgas zum Verdampfer zurückgeführt werden.

## Katalysatoren

Als Katalysatoren wurden Nickel, Ruthenium, Rhodium und Palladium oder deren Kombinationen eingesetzt, die auf Lithium-Aluminium-Spinell als Katalysatorträger aufgebracht worden waren.

Die Herstellung des Katalysator-Trägers sei beispielhaft geschildert: 2,86 Liter kugelförmiges $\gamma$-Aluminiumoxid mit 1—3 mm bzw. 4—6 mm Durchmesser und einer inneren Oberfläche von ca. 250 m²/g wurden mit 1 Liter wäßriger Lösung bei 30°C getränkt, in die nacheinander 296 g Ameisensäure und 233 g 54%ige wäßrige Lithiumhydroxidlösung gegeben worden waren. Das getränkte Aluminiumoxid wurde bei 150°C im Vakuum getrocknet, mit der gleichen Lösung nochmals getränkt und wieder bei 150°C im Vakuum getrocknet. Der Träger wurde anschließend 6 Std. bei 1050°C zum Lithium-Aluminium-Spinell geglüht, was durch eine Röntgenaufnahme bestätigt wurde (60% Spinell). Der fertige Träger hatte eine innere Oberfläche von ca. 30 m²/g und eine mittlere Porenweite von ca. 700 Å.

Das Aufbringen der (Edel)Metalle erfolgte in der herkömmlichen Art durch Tränken des Trägers mit der wäßrigen Metallsalzlösung, Reduktion des Metallsalzes, Auswaschen der Anionen und Trocknen. Auf diese Weise wurden die folgenden Träger-Katalysatoren mit den angegebenen Metallgehalten hergestellt:

| Katalysator-Nr. | % (Edel)Metall | Katalysator-Nr. | % (Edel)Metall |
|---|---|---|---|
| 1 | 1 % Ru | 5 | 0,5 % Ru 0.5 % Pd |
| 2 | 1 % Rh | 6 | 0,5 % Rh 0,5 % Pd |
| 3 | 1 % Pd | 7 | 0,3 % Ru 0,3 % Rh |
| 4 | 0,5 % Ru 0,5 % Rh | | 0,3 % Pd |
| | | 8 | 5 % Ni |

## Durchführung der Hydrierung

Der Reaktor wurde mit 3 l des Katalysators gefüllt. Es wurde so viel der zu hydrierenden Komponente eingesetzt, daß die Raum-Zeit-Ausbeute zwischen 0,1—2 kg betrug. Im einzelnen betrug die Raum-Zeit-Ausbeute bei den in der folgenden Tabelle aufgeführten Versuchen:

| | |
|---|---|
| Versuch 2 | = 2,0 kg/l × h |
| Versuch 4 | = 1,0 kg/l × h |
| Versuch 1, 3 und 9 | = 0,5 kg/l × h |
| Versuch 5—8, 10—11 | = 0,3 kg/l × h |
| Versuch 12—19 | = 0,1—0,2 kg/l × h |

**0 005 737**

Die Verdünnung des Einsatzproduktes mit dem Hydrierprodukt betrug bei:

| | |
|---|---|
| Versuch 1 | = 1:100 |
| Versuch 2—5 | = 1:30 |
| Versuch 6—11 | = 1:15 |
| Versuch 12—18 | = 1:10 |
| Versuch 19 | = 1:1 |

Die Hydrierung erfolgte bei den Versuchen 1 und 3 in der Gasphase, bei den Versuchen 13, 16 und 18 in der Flutphase, bei allen anderen in der Rieselphase.

5

Hydrierbeispiele

Die Beispiele für die Hydrierung der verschiedenen aromatischen Carbonsäureester zu den entsprechenden cycloaliphatischen Estern sind in der folgenden Tabelle zusammengefaßt

| Versuchs-Nr. | Einsatzprodukt | Hydrierprodukt | Katalysator-Nr. | Temp. °C | Druck bar | % Ausbeute der Theorie | Siedepunkt des Hydrierprodukt °C /mm Hg |
|---|---|---|---|---|---|---|---|
| 1 | Benzoesäure-methylester | Hexahydrobenzoe-säuremethylester | 8 | 120 | 50 | 99 | 182—83 °C /750 |
| 2 | Benzoesäure-isopropylester | Hexahydrobenzoe-säureisopropylester | 3 | 130 | 60 | 98,4 | 76—77 °C / 12 |
| 3 | Benzoesäure-äthylester | Hexahydrobenzoe-säureäthylester | 6 | 120 | 50 | 99 | 199—200 °C /750 |
| 4 | Benzoesäure-propylester | Hexahydrobenzoe-säurepropylester | 4 | 120 | 50 | 98,9 | 216 °C /750 |
| 5 | Benzoesäure-isoamylester | Hexahydrobenzoe-säureisoamylester | 5 | 140 | 50 | 98,7 | 258 °C /750 |
| 6 | o-Toluylsäure-methylester | Hexahydro-o-toluyl säuremethylester | 4 | 120 | 70 | 99 | 189 °C /760 |
| 7 | o-Toluylsäure-äthylester | Hexahydro-o-toluyl-säureäthylester | 6 | 130 | 70 | 99 | 204 °C /750 |
| 8 | m-Toluylsäure-äthylester | Hexahydro-m-toluyl-säureäthylester | 5 | 130 | 70 | 98,4 | 210 °C /760 |
| 9 | m-Toluylsäure-methylester | Hexahydro-m-toluyl-säuremethylester | 3 | 140 | 70 | 98,6 | 196—97 /750 |

Hydrierbeispiele (Fortsetzung)

| Versuchs-Nr. | Einsatzprodukt | Hydrierprodukt | Katalysator-Nr. | Temp. °C | Druck bar | % Ausbeute der Theorie | Siedepunkt des Hydrierprodukt °C /mm Hg |
|---|---|---|---|---|---|---|---|
| 10 | p-Toluylsäure-methylester | Hexahydro-p-toluyl-säuremethylester | 2 | 145 | 70 | 98,7 | 198°C /750 |
| 11 | p-Toluylsäure-äthylester | Hexahydro-p-toluyl-säureäthylester | 1 | 150 | 75 | 98,5 | 209°C /750 |
| 12 | Homophthalsäure-dimethylester | Hexahydrohomophthal-säuredimethylester | 7 | 160 | 70 | 97,6 | 143−45°C /20 |
| 13 | Homophthalsäure-diäthylester | Hexahydrohomophthal-säurediäthylester | 5 | 160 | 70 | 97,8 | 276°C /760 |
| 14 | o-Phthalsäure-dimethylester | Hexahydrophthal-säuredimethylester | 1 | 140 | 100 | 99 | 128°/20 |
| 15 | o-Phthalsäure-diäthylester | Hexahydrophthal-säurediäthylester | 4 | 140 | 100 | 99 | 143°C /20 |
| 16 | Isophthalsäure-dimethylester | Hexahydroisophthal-säuredimethylester | 1 | 150 | 100 | 99 | 140°C /20 |
| 17 | Isophthalsäure-diäthylester | Hexahydroisophthal-säurediäthylester | 2 | 160 | 100 | 98,8 | 290°C /760 |
| 18 | Terephthalsäure-dimethylester | Hexahydroterephthal-säuredimethylester | 1 | 180 | 100 | 97,8 | 150°C /20 |
| 19 | Terephthalsäure-diäthylester | Hexahydroterephthal-säurediäthylester | 1 | 180 | 100 | 98,5 | 110°C /1 |

**Patentansprüche**

Verfahren zur kontinuierlichen katalytischen Hydrierung von aromatischen Carbonsäureestern zu den entsprechenden cycloaliphatischen Carbonsäureestern, gegebenenfalls in Gegenwart des Hydrierproduktes als Verdünnungsmittel, in der Riesel- oder Flutphase bei Temperaturen von 70°C bis 250°C und Drucken von 30 bar bis 200 bar bzw. in der Gasphase bei 70°C bis 250°C und 1 bar bis 10 bar in Gegenwart von mindestens einem, auf Trägern aufgebrachten Katalysator aus der Reihe Nickel, Ruthenium, Rhodium oder Palladium im Festbett, dadurch gekennzeichnet, daß man als Träger für die Katalysatoren ein Aluminiumoxid verwendet, das zu mindestens 20% in Lithium-Aluminium-Spinell umgewandelt worden ist.

**Revendication**

Procédé d'hydrogénation catalytique continu d'esters d'acides carboxyliques aromatiques en esters d'acides carboxyliques cycloaliphatiques correspondants, éventuellement en présence du produit hyrogéné comme diluant, en phase de ruissellement ou en nappe à des températures de 70 à 250°C et à des pressions de 30 à 200 bars (3 à 20 MPa) ou en phase gazeuse à 70—250°C et sous pression de I à 10 bars (0,1 à I MPa) en présence d'au moins un catalyseur fixé sur des supports, de la série du nickel, du ruthénium, du rhodium ou du palladium en lit fixe, caractérisé en ce qu'on utilise comme support pour les catalyseur un oxyde d'aluminium qui a été transformé au moins à 20% en spinelle de lithium et d'aluminium.

**Claim**

Process for the continuous catalytic hydrogenation of aromatic carboxylic acid esters to form the corresponding cycloaliphatic carboxylic acid ester, optionally in the presence of the hydrogenation product as diluent, in the trickle or flood phase at temperatures of 70°C to 250°C and pressures of 30 bar to 200 bar, or in the gas phase at 70°C to 250°C and 1 bar to 10 bar in the presence of at least one supported catalyst from the series comprising nickel, ruthenium, rhodium or palladium in the form of a fixed bed, characterised in that the support used for the catalysts is an aluminium oxide of which at least 20% has been converted into lithium-aluminium spinel.